(19) Europäisches Patentamt
European Patent Office
Office européen des brevets

(11) **EP 1 405 651 A1**

(12) **DEMANDE DE BREVET EUROPEEN**

(43) Date de publication:
**07.04.2004 Bulletin 2004/15**

(51) Int Cl.$^7$: **A61M 35/00**

(21) Numéro de dépôt: **03292399.7**

(22) Date de dépôt: **29.09.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **01.10.2002 FR 0212157**

(71) Demandeur: **L'OREAL**
**75008 Paris (FR)**

(72) Inventeurs:
• **Hirt, Pascal**
**92210 Saint-Cloud (FR)**
• **Jourdain, Roland**
**92360 Meudon La Fôret (FR)**

(74) Mandataire: **Tanty, François et al**
**Nony & Associés,**
**3, rue de Penthièvre**
**75008 Paris (FR)**

(54) **Kit d'évaluation ou de diagnostic**

(57) La présente invention concerne un kit (10) d'évaluation ou de diagnostic, comportant une pluralité d'applicateurs (20) contenant des produits de test de natures différentes et/ou comportant au moins un composé à des concentrations différentes, chaque applicateur (20) comportant :

- un tube (21),
- un bouchon d'un liquide ou d'une poudre (24) à l'intérieur du tube (21), et
- au moins un produit de test contenu dans un espace intérieur du tube (21) délimité d'un premier côté par le bouchon de liquide ou de poudre (24), ce bouchon étant agencé pour s'évacuer avec le produit lorsque ce dernier quitte l'espace intérieur du tube lors de l'utilisation.

FIG.1

**Description**

La présente invention concerne les kits de diagnostic ou d'évaluation, et notamment mais non exclusivement ceux destinés à permettre d'évaluer le niveau de neurosensibilité cutanée d'un individu ou sa sensibilité à un allergène.

On connaît par US 5 143 210 un kit de diagnostic comportant un support pouvant recevoir plusieurs flacons contenant chacun un produit de test. Chaque flacon est relativement coûteux à réaliser et l'application d'un produit de test demande de nombreuses manipulations. Un flacon peut être renversé accidentellement lors de son maniement. Un excès de produit est susceptible d'être prélevé.

Il existe un besoin pour disposer d'un kit de diagnostic ou d'évaluation facile à transporter, capable d'être fabriqué à un coût relativement faible et simple à utiliser.

On connaît par US 3 958 571 un applicateur comportant un tube comportant un liquide et un élément d'application à une extrémité du tube. Un tel applicateur est prévu pour appliquer un médicament tel qu'une solution d'iode.

L'invention a pour objet, selon l'un de ses aspects, un kit de diagnostic ou d'évaluation comportant une pluralité d'applicateurs contenant des produits de test de natures différentes et/ou comportant au moins un composé à des concentrations différentes, chaque applicateur comportant :

- un tube,
- un bouchon d'un liquide ou d'une poudre à l'intérieur du tube, et
- au moins un produit de test contenu dans un espace intérieur du tube délimité d'un premier côté par le bouchon de liquide ou de poudre, ce bouchon étant agencé pour s'évacuer avec le produit de test lorsque ce dernier quitte l'espace intérieur du tube lors de l'utilisation.

Par « produit de test », on désigne un produit servant à caractériser, à lui seul ou en combinaison avec d'autres produits ou réactifs, un état d'un individu.

Grâce à l'invention, il est possible d'appliquer, sur la peau y compris les muqueuses ou sur les phanères par exemple, différentes concentrations d'un composé ou différents produits sans les inconvénients liés à l'utilisation de flacons.

De plus, la quantité de produit de test contenue dans chaque applicateur peut aisément être réduite à celle juste nécessaire pour l'application visée, en choisissant de manière appropriée les dimensions du tube. Le volume de liquide contenu dans le tube peut ainsi être compris par exemple entre 0,01 ml et 5 ml, mieux entre 0,05 ml et 1 ml.

L'invention permet également de réaliser un kit de diagnostic ou d'évaluation avec un coût compatible avec une large diffusion.

Dans un exemple de mise en oeuvre de l'invention, le kit peut comporter une pluralité d'applicateurs contenant des produits comportant au moins un composé à des concentrations différentes.

Le kit peut par exemple comporter au moins deux produits de test comportant au moins un composé avec des concentrations variant d'un facteur compris entre 1,5 et 10, par exemple entre 2 et 5, d'un applicateur à l'autre.

Les produits de test contenus dans les différents applicateurs peuvent contenir un agent de stimulation du système nerveux périphérique, par exemple.

Par « agent de stimulation du système nerveux périphérique », on désigne un composé qui permet d'induire une réponse sensorielle liée à la mise en jeu de nerfs sensitifs et cutanés, dont les terminaisons affleurent au niveau du stratum corneum.

L'agent de stimulation du système nerveux périphérique peut être choisi parmi les capsaïcinoids naturels ou synthétiques, de préférence la capsaïcine, l'homocapsaïcine, l'homodihydrocapsaïcine, la nordihydrocapsaïcine, la dihydrocapsaïcine ; l'acide lactique ; l'acide glycolique ; l'éthanol à une concentration supérieure à 50% ; l'huile de moutarde, cette liste n'étant pas limitative. On pourra utilement se référer au brevet européen EP 680 749.

La concentration dans chaque produit de test en agent de stimulation du système nerveux périphérique peut être comprise, par exemple, entre $10^{-6}$ et $10^{-2}$ % en poids, par rapport au poids total de la composition.

Le kit peut comporter au moins un produit de test dépourvu d'agent de stimulation du système nerveux périphérique, afin par exemple de permettre à un sujet subissant un test de comparer les sensations ressenties dans le cas de l'application de deux produits de test dont seul l'un contient l'agent de stimulation du système nerveux périphérique.

Dans un autre exemple de mise en oeuvre de l'invention, le kit peut comporter une pluralité d'applicateurs contenant des produits de test de natures différentes. Les produits de test peuvent par exemple contenir des allergènes respectifs de natures différentes.

Les allergènes peuvent par exemple être choisis dans le groupe constitué par : les allergènes issus des acariens, des poils et squames d'animaux, des moisissures, des venins d'hyménoptères, des aliments, des métaux, notamment du nickel, du caoutchouc, de tout composé pouvant être rencontré dans des produits destinés à être appliqués sur le corps ou les cheveux, par exemple un composé utilisé dans les produits de coloration capillaire, notamment du PPD (Paraphényldiamine), cette liste n'étant pas limitative.

Les applicateurs peuvent être conditionnés de diverses manières.

Le kit peut comporter par exemple un boîtier comportant des compartiments dans lesquels sont logés les applicateurs.

Le boîtier peut notamment comporter au moins un compartiment configuré pour recevoir un applicateur unique, ou en variante, le boîtier peut comporter au moins un compartiment configuré pour recevoir une pluralité d'applicateurs.

Dans une autre variante encore, le kit peut comporter au moins un sachet de conditionnement d'au moins un applicateur, voire un chapelet de sachets contenant chacun au moins un applicateur.

Chaque applicateur peut comporter au moins une indication représentative de la nature du produit contenu à l'intérieur du tube et/ou de la concentration d'un composé contenu dans le produit, par exemple au moins un signe alphanumérique et/ou au moins une couleur.

L'espace intérieur du tube dans lequel est contenu le produit de test peut être délimité d'un second côté, opposé au premier, par une partie sécable, amovible, perforable ou déformable.

Lorsque la partie sécable est rompue, le produit contenu dans l'espace intérieur du tube peut descendre par gravité, chassant le bouchon de liquide ou de poudre.

L'applicateur peut être agencé de telle sorte qu'après rupture de l'extrémité sécable, l'utilisateur puisse doser la quantité de liquide qui descend en manipulant le tube comme une pipette dont il obture l'extrémité supérieure avec l'index, le tube pouvant être incliné plus ou moins, le cas échéant.

Dans un exemple de mise en oeuvre de l'invention, le tube peut être rebouché après qu'une fraction seulement du liquide contenu à l'intérieur soit descendue. Ce rebouchage peut s'effectuer par exemple grâce à l'extrémité sécable. Cette dernière peut être configurée par exemple pour pouvoir constituer un bouchon de fermeture, l'extrémité sécable comportant par exemple un picot apte à s'engager dans le tube ou sur le tube pour l'obturer.

L'applicateur peut comporter, le cas échéant, un élément de maintien de la partie sécable sur l'applicateur après son sectionnement.

Le tube peut être muni à une extrémité d'un élément d'application, cet élément d'application étant séparé du produit de test, avant l'utilisation de l'applicateur, par le bouchon de liquide ou de poudre.

L'élément d'application peut être poreux, par exemple fibreux, afin par exemple d'être aisément imprégné par le produit de test.

L'élément d'application peut être choisi dans le groupe constitué par : un embout en coton, un embout en mousse, une pointe feutre, un embout floqué, un embout en céramique ou en un matériau fritté, cette liste n'étant pas limitative.

En variante, le tube peut être dépourvu d'élément d'application. Dans ce cas, le tube peut par exemple comporter une extrémité configurée pour permettre d'effectuer une scarification de la peau.

Le bouchon peut comporter, comme indiqué plus haut, un liquide et/ou une poudre.

Lorsque le bouchon comporte un liquide, ce dernier peut être choisi par exemple dans le groupe constitué par : les huiles minérales, les produits fluorés, les silicones, cette liste n'étant pas limitative.

Lorsque le bouchon comporte une poudre, cette dernière peut comporter des particules organiques ou minérales, pleines ou creuses, et être choisie par exemple dans le groupe constitué par : les poudres de microsphères de copolymères telles que l'Expancel® (Nobel Industrie), de Nylon® (notamment l'Orgasol®, des cires, des silices et des silicones, cette liste n'étant pas limitative.

Le tube peut être réalisé dans une matière transparente, notamment une matière plastique transparente, afin de permettre par exemple à l'utilisateur d'observer le niveau de produit dans le tube ou sa couleur.

Le tube peut comporter une structure multicouche, avec au moins une couche formant une barrière vis-à-vis de l'air, par exemple une couche de vernis imperméable à l'air ou à un solvant ou anti-UV.

Le produit de test est avantageusement stérile.

L'ensemble des applicateurs du kit peut avoir été stérilisé dans sa totalité.

L'invention a encore pour objet, selon un autre de ses aspects, un procédé d'évaluation du niveau de neurosensibilité cutanée d'un individu, pouvant comporter les étapes suivantes :

1) appliquer sur une zone cutanée de l'individu un premier produit contenant un véhicule physiologiquement acceptable et un agent de stimulation du système nerveux périphérique, le produit étant appliqué en utilisant un applicateur comportant un tube, un bouchon d'un liquide ou d'une poudre à l'intérieur du tube, le produit étant contenu dans un espace intérieur du tube délimité d'un premier côté par le bouchon de liquide ou de poudre, ce bouchon étant agencé pour s'évacuer avec le produit lorsque ce dernier quitte l'espace intérieur du tube lors de l'utilisation,

2) recueillir une information représentative de la détection par l'individu d'une sensation dysesthésique,

3) en l'absence de sensation détectée par l'individu, recommencer les étapes 1) et 2) avec un produit contenant une concentration plus élevée du même agent jusqu'à ce que l'individu détecte une sensation dysesthésique ou jusqu'à ce qu'un produit ayant une valeur maximale de concentration dudit agent soit appliqué,

4) déduire, de la dernière concentration appliquée, une information quant à la neurosensibilité cutanée de l'individu.

Par « véhicule physiologiquement acceptable », on désigne un véhicule compatible avec la peau, les muqueuses, les ongles, les cheveux. Il peut s'agir par exemple d'une solution aqueuse, hydroalcoolique, ou huileuse.

La zone cutanée choisie peut être le pli du bras, le lobe de l'oreille ou le visage, en particulier l'aile du nez, le sillon naso-génien ou l'angle du maxillaire inférieur.

Une sensation dysesthésique peut être la plus petite sensation non douloureuse ressentie dans une zone traitée par l'agent de stimulation.

Un produit cosmétique ou de soin peut être prescrit en fonction de la neurosensibilité cutanée de l'individu. Par « produit cosmétique », on désigne un produit tel que défini dans la Directive 93/35/CE du Conseil du 14 juin 1993, modifiant pour la sixième fois la directive 76/768/CEE.

L'information déduite de la dernière concentration appliquée peut être transmise à distance, par le réseau Internet notamment.

L'invention a encore pour objet, selon un autre de ses aspects, un procédé de diagnostic d'une allergie, comportant les étapes suivantes :

- déposer un produit contenant un allergène sur la peau ou une muqueuse d'un individu, le produit étant déposé à l'aide d'un applicateur comportant un tube, un bouchon d'un liquide ou d'une poudre à l'intérieur du tube, le produit étant contenu dans un espace intérieur du tube délimité d'un premier côté par le bouchon de liquide ou de poudre, ce bouchon étant agencé pour s'évacuer avec le produit lorsque ce dernier quitte l'espace intérieur du tube lors de l'utilisation,
- déduire d'une réaction cutanée éventuelle de l'individu une information concernant la sensibilité de l'individu à l'allergène considéré.

L'invention a encore pour objet, selon un autre de ses aspects, un procédé d'évaluation d'un produit de traitement, comportant les étapes suivantes :

- réaliser les étapes d'un des procédés d'évaluation ou de diagnostic définis précédemment,
- appliquer le produit de traitement,
- après un nombre d'applications donné et/ou qu'une durée déterminée se soit écoulée, réaliser à nouveau les étapes du procédé d'évaluation ou de diagnostic,
- évaluer l'efficacité du traitement suivi.

L'invention a encore pour objet, indépendamment ou en combinaison avec ce qui précède, un procédé d'application d'au moins un produit contenu dans un espace intérieur du tube dans lequel on soumet le tube à une source de chaleur préalablement à l'application du produit. Ce produit peut comporter par exemple au moins un épaississant thermoréversible de telle sorte que le produit se fluidifie lorsque la chaleur augmente.

La source de chaleur peut par exemple être la chaleur humaine, ou une source de chaleur externe au corps humain, par exemple. Le fait de fluidifier le produit préalablement à l'application peut permettre d'améliorer sa conservation pendant le stockage dans le tube, notamment de limiter son évaporation. Le fait de fluidifier le produit peut également faciliter son passage à travers un embout d'application, notamment un embout réalisé dans un matériau poreux, par exemple en coton. Le tube peut comporter ou non un bouchon d'un liquide ou d'une poudre disposé d'un côté du produit, ce bouchon de liquide ou de poudre pouvant s'évacuer avec le produit lorsque celui-ci quitte l'espace intérieur du tube lors de l'utilisation.

L'invention pourra être mieux comprise à la lecture de la description détaillée qui va suivre, d'exemples de mise en oeuvre non limitatifs de celle-ci, et à l'examen du dessin annexé, sur lequel :

- la figure 1 représente, de manière schématique, un kit de diagnostic ou d'évaluation conforme à un exemple de mise en oeuvre de l'invention,
- les figures 2 à 4 représentent isolément un applicateur du kit de la figure 1,
- les figures 5 et 6 représentent d'autres exemples de kits selon l'invention,
- la figure 7 représente un applicateur conditionné dans un sachet individuel,
- les figures 8 à 11 illustrent différentes variantes de réalisation de l'élément d'application,
- la figure 12 représente un applicateur dépourvu d'élément d'application,
- la figure 13 représente de manière partielle et schématique une variante de l'applicateur de la figure 12, et
- les figures 14 et 15 représentent des variantes de réalisation de la partie sécable du tube,
- les figures 16 et 17 représentent respectivement, de manière schématique, des applicateurs comportant des compositions bi-phasiques et multiphasiques,
- la figure 18 représente, de manière schématique et partielle, un autre exemple d'applicateur,
- la figure 19 représente un récipient pouvant recevoir un applicateur, et
- la figure 20 représente un autre exemple de porte-applicateur.

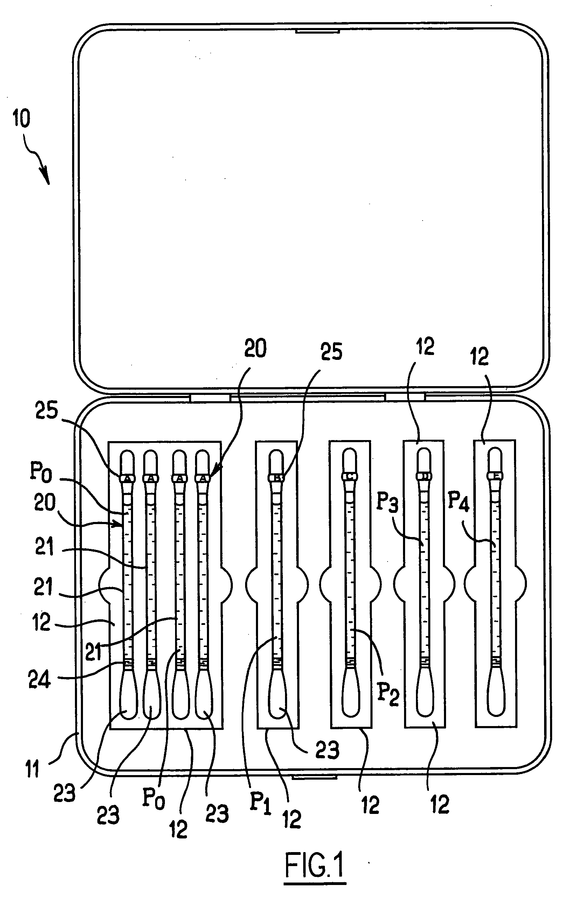

On a représenté, à la figure 1, un exemple d'un kit 10 de diagnostic ou d'évaluation réalisé conformément à l'invention.

Ce kit 10 peut comporter un boîtier 11 comportant une pluralité de compartiments 12 recevant chacun un applicateur 20 contenant un produit de test. Il peut s'agir, par exemple, de produits permettant de tester la neurosensibilité cutanée d'un individu.

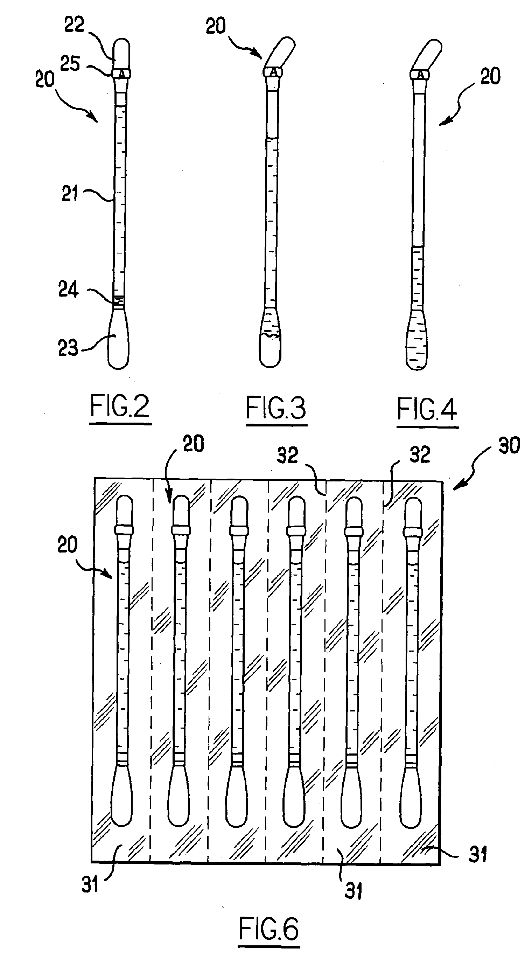

On a représenté isolément, aux figures 2 à 4, un applicateur 20.

Celui-ci peut être, par exemple, un applicateur tel que décrit dans le brevet US 5 702 035 dont le contenu est incorporé ici par référence. Des applicateurs de ce type sont commercialisés par la société californienne Swabplus Inc.

Chaque applicateur 20 comporte un tube 21 contenant le produit de test, ce tube étant, dans l'ensemble illustré, réalisé par extrusion de matière plastique transparente et pourvu à une extrémité fermée d'une partie sécable 22. Cette dernière est recouverte, dans l'exemple considéré, par un embout de coton. Le tube 21 est ouvert à l'autre extrémité opposée à la partie sécable 22, étant muni par exemple à cette extrémité d'un élément d'application 23.

Dans l'exemple illustré, l'élément d'application 23 comporte un embout de coton, à la manière d'un coton-tige.

Le produit de test est contenu dans un espace intérieur du tube 21 situé entre la partie sécable 22 et un bouchon 24 présent dans le tube 21, du côté de son extrémité ouverte.

Le volume de produit de test peut convenir à un usage unique de l'applicateur, étant déterminé en fonction de la nature du produit et de l'application envisagée, et pouvant varier par exemple entre 0,01 ml et 5 ml, mieux entre 0,05 et 1 ml. Le diamètre extérieur du tube 21 est par exemple inférieur à 6 mm, voire inférieur à 3 mm environ. Le diamètre intérieur du tube 21 peut être compris entre environ 0,5 mm et environ 3 mm, par exemple.

Le bouchon 24 peut être constitué par tout liquide ou poudre inerte compatible avec le conditionnement du produit dans le tube 21, notamment un liquide ne réagissant pas avec le produit de test, capable d'être évacué facilement hors du tube 21 au moment de l'utilisation, et par ailleurs physiologiquement acceptable. Le bouchon 24 permet notamment d'isoler le produit de test de l'air, d'empêcher son évaporation et à des contaminants extérieurs de pénétrer. Le liquide du bouchon de liquide 24 peut être constitué, par exemple, par une huile minérale ou un produit fluoré, entre autres. Dans l'exemple considéré, le bouchon de liquide 24 est formé de silicone.

La quantité de liquide ou de poudre formant le bouchon 24 est faible par rapport à celle du produit de test.

Lorsque la partie sécable 22 est rompue, l'air peut pénétrer dans le tube 21 du côté opposé à l'extrémité ouverte et le produit peut s'écouler par gravité dans le tube 21 et gagner l'élément d'application 23, comme illustré sur les figures 3 et 4, pour être appliqué sur la peau par exemple.

Dans l'exemple considéré, l'embout de coton recouvrant la partie sécable 22 permet de maintenir celle-ci solidaire du reste du tube 21, même après sa rupture.

On peut voir sur les figures 1 à 4 que chaque applicateur 20 comporte une indication 25 visible par l'utilisateur, cette indication 25 pouvant comporter, par exemple, un signe alphanumérique ou une couleur spécifique permettant à l'utilisateur de distinguer les différents applicateurs entre eux.

Le kit 10 peut par exemple comporter une pluralité d'applicateurs 20 comportant respectivement des produits $P_0$, $P_1$, $P_2$, $P_3$ et $P_4$ différents, associés à des indications 25 respectives. Dans l'exemple de la figure 1, le boîtier contient huit applicateurs 20 mais il ne s'agit que d'un exemple et le nombre d'applicateurs peut varier largement sans que l'on sorte du cadre de la présente invention.

Les produits $P_0$ à $P_4$ peuvent être de natures différentes ou de même nature. Dans ce dernier cas, les produits $P_0$ à $P_4$ peuvent contenir un même composé mais à des concentrations respectives différentes.

Le kit selon l'invention peut comprendre en outre une crème émoliante pour traiter la zone d'application, non représentée sur les figures, un mode d'emploi du kit et une notice contenant des conseils, le cas échéant.

Le kit 10 peut être utilisé par exemple, comme mentionné plus haut, pour évaluer la neurosensibilité cutanée d'un individu.

Les différents applicateurs 20 peuvent alors contenir de la capsaïcine à des concentrations différentes.

Par exemple, les produits $P_0$, $P_1$, $P_2$, $P_3$, $P_4$ peuvent contenir de la capsaïcine en solution hydroalcoolique (éthanol 10 % eau 90 %) à des concentrations massiques respectives croissantes, égales par exemple aux valeurs suivantes : $C_0 = 0$ % (véhicule seul), $C_1 = 3,3.10^{-5}$ %, $C_2 = 1.10^{-4}$ %, $C_3 = 3,3.10^{-4}$ %, $C_4 = 1.10^{-3}$ %.

Pour effectuer un diagnostic, on peut par exemple procéder de la manière suivante.

1ère étape: On applique sur les sillons nasogéniens, de chaque côté du visage, du produit de test de concentration $C_0$ pour habituer le sujet aux sensations induites par l'application du véhicule. On attend ensuite 3 minutes.

2ème étape: Application du véhicule seul d'un côté et de la solution de capsaïcine à la concentration $C_1$ de l'autre. Au bout de 3 minutes, on demande au sujet s'il a senti une différence entre les deux côtés. Si le sujet décrit une sensation dysesthésique du côté ayant reçu la capsaïcine, on arrête le test et l'on attribue $C_1$ comme seuil de détection. Sinon, on va à l'étape suivante.

3ème étape: Application de la solution $C_2$ du côté ayant reçu le véhicule à l'étape 1, et du véhicule seul de l'autre côté. Au bout de 3 minutes, on demande si le sujet a senti une différence entre les deux côtés. Si le sujet décrit une sensation particulière du côté ayant reçu la capsaïcine, on arrête le test et l'on attribue $C_2$ comme seuil de détection. Sinon, on va à l'étape 4.

4ème étape : Application de la solution $C_3$ du côté ayant reçu le véhicule à l'étape 2, et du véhicule seul de l'autre côté. Au bout de 3 minutes, on demande si le sujet a senti une différence entre les deux côtés. Si le sujet décrit une sensation particulière du côté ayant reçu la capsaïcine, on arrête le test et l'on attribue $C_3$ comme seuil de détection. Sinon, on va à l'étape 5.

5ème étape: Application de la solution $C_4$ du côté ayant reçu le véhicule seul à l'étape 3 et du véhicule seul de l'autre côté. Au bout de 3 minutes, on demande si le sujet a senti une différence entre les deux côtés. Si le sujet décrit une sensation particulière du côté ayant reçu la capsaïcine, on attribue $C_4$ comme seuil de détection. Sinon, on attribue comme seuil de détection une concentration supérieure à $C_4$.

Si l'individu ne détecte aucune sensation dysesthésique après l'application de tous les applicateurs 20, l'individu est déclaré à peau peu ou pas sensible. S'il détecte une sensation dysesthésique après l'application du produit $P_1$, $P_2$ ou $P_3$, l'individu est déclaré respectivement à peau excessivement sensible, peau très sensible, ou peau moyennement sensible.

On peut considérer que l'individu a une neurosensibilité très élevée si la concentration en capsaïcine du produit détecté par l'individu est comprise entre $1.10^{-6}$ % et $1.10^{-4}$ %, moyennement élevée si elle est comprise entre $1,5.10^{-4}$ % et $0,75.10^{-3}$ %, faible si elle est comprise entre $1.10^{-3}$ % et $5.10^{-3}$ %, et très faible si elle est de l'ordre de $1.10^{-2}$%.

On remarquera qu'entre deux produits $P_1$, $P_2$, $P_3$ ou $P_4$ successifs, la concentration augmente d'un facteur d'environ $\sqrt{10}$, mais elle peut augmenter d'un facteur autre sans que l'on sorte du cadre de la présente invention, par exemple d'un facteur compris entre 1,5 et 10, de préférence entre 2 et 5.

Bien entendu, les applicateurs 20 peuvent être conditionnés autrement.

On a représenté, à la figure 5, une variante du kit de la figure 1 dans laquelle le boîtier 11 comporte une pluralité de compartiments 17 capables de recevoir chacun plusieurs applicateurs 20 comportant un produit de test identique.

Le boîtier peut comporter un repère 18 associé à chaque compartiment 17 pour permettre à l'utilisateur d'identifier la nature du produit contenu dans les applicateurs présents dans le compartiment 17 correspondant.

Les applicateurs 20 peuvent être conditionnés autrement que dans un boîtier.

A titre d'exemple, on a représenté à la figure 6 un kit comportant un chapelet 30 de sachets 31 contenant par exemple chacun un applicateur 20. Chaque applicateur peut être stérile.

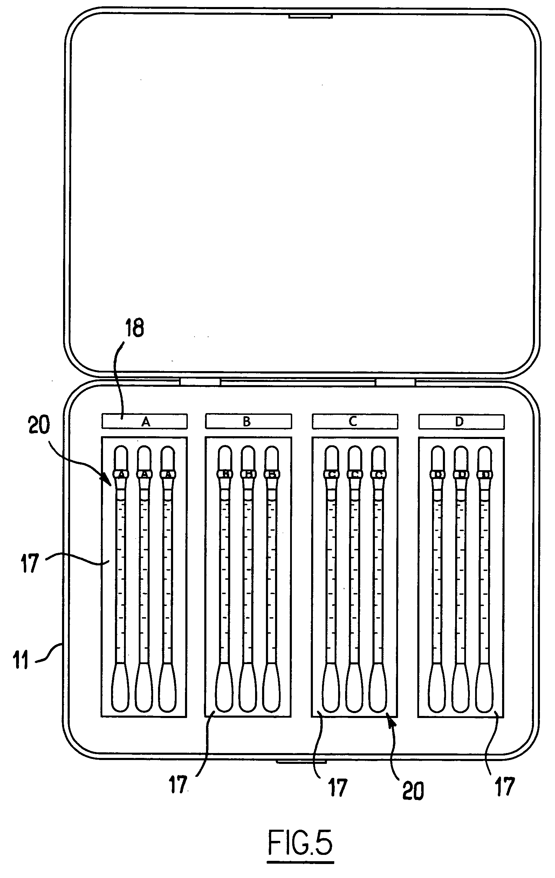

Les applicateurs 20 peuvent encore être conditionnés dans des sachets individuels 33, comme illustré à la figure 7.

Chaque applicateur 20 peut, le cas échéant, comporter un élément d'application pré-imprégné par un composé 34 particulaire ou par un liquide avant l'utilisation.

Lorsque l'élément d'application est pré-imprégné par un composé 34 liquide, l'applicateur 20 est avantageusement proposé dans un emballage étanche tel que par exemple le sachet représenté à la figure 7.

Le produit contenu dans le tube 21, en s'écoulant dans l'élément d'application, peut dissoudre le composé 34 qui imprègne l'élément d'application 23 ou réagir avec celui-ci. Cela permet par exemple de disposer d'un applicateur comportant au moins deux composés ne pouvant être conditionnés ensemble et devant être mélangés extemporanément.

Un applicateur 20 peut comporter un élément d'application qui se présente sous une forme autre qu'un embout de coton conventionnel.

L'élément d'application peut par exemple avoir une forme effilée, comme représenté à la figure 8.

L'élément d'application peut encore comporter, par exemple, tout matériau poreux, par exemple fibreux, élastiquement compressible ou non.

A titre d'exemple, on a représenté à la figure 9 un élément d'application qui se présente sous la forme d'un embout en mousse 35.

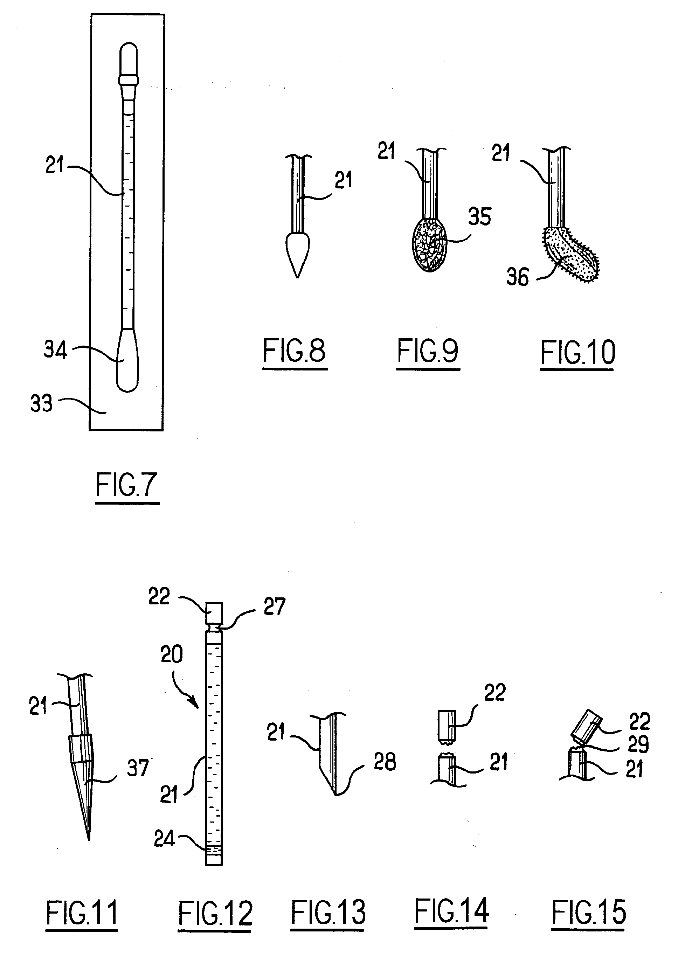

L'élément d'application peut comporter un flocage 36 à sa surface, comme illustré à la figure 10. On voit également sur cette figure que l'élément d'application peut avoir une forme incurvée, avec une partie s'étendant selon un axe longitudinal non confondu avec l'axe du tube 21.

L'élément d'application peut encore se présenter, par exemple, sous la forme d'un embout 37 ayant une forme effilée, notamment une pointe feutre comme illustré sur la figure 11.

L'embout peut être réalisé dans une matière poreuse ou, en variante, dans une matière non poreuse, mais comporter au moins un canal intérieur ou une rainure permettant au produit de test contenu dans le tube 21 de s'écouler vers l'extrémité distale.

L'applicateur 20 peut encore être dépourvu d'élément d'application, comme représenté à la figure 12. Une telle réalisation peut être utile notamment lorsque le produit contenu dans le tube 21 doit être déposé sous la forme d'une goutte à la surface de la peau, par exemple.

On peut encore réaliser le tube 21 avec une forme effilée du côté opposé à la partie sécable 22, par exemple avec une forme biseautée, comme illustré sur la figure 13.

On peut ainsi former, à l'extrémité du tube 21, une pointe 28 permettant d'effectuer une légère scarification de la peau. Une telle scarification peut être utile, si l'on cherche à évaluer la sensibilité d'un individu à différents allergènes en effectuant une cuti-réaction.

Dans ce cas, on peut effectuer, au moyen de la pointe 28, une légère scarification de la peau puis casser la partie sécable 22 afin de permettre au produit de test contenu à l'intérieur du tube 21 de s'écouler sur la zone de peau comportant la scarification.

Une réaction cutanée observée après quelques minutes ou quelques heures, voire plusieurs jours, peut donner une information sur la sensibilité de l'individu à l'allergène considéré.

On peut encore, en variante, si la forme de l'extrémité de l'applicateur le permet, utiliser celui-ci pour effectuer un prick-test en déposant tout d'abord sur la peau le produit de test contenu dans le tube 21 puis en piquant avec la pointe 28 la peau à travers le produit déposé à sa surface, afin de faciliter sa pénétration dans le derme.

Le produit de test contenu dans les applicateurs 20 peut renfermer différents allergènes. Il peut notamment permettre de connaître la sensibilité d'un individu à des allergènes tels que ceux issus des acariens, des poils et squames d'animaux, des moisissures, des venins d'hyménoptères, des aliments, des métaux, du caoutchouc, cette liste n'étant pas limitative.

On voit sur la figure 12 que la partie sécable peut être reliée au reste du tube par une zone de cassure préférentielle 27 matérialisée par exemple par un amincissement de la paroi du tube 21 à ce niveau.

La partie sécable peut être réalisée de différentes autres manières sans que l'on sorte du cadre de la présente invention.

On peut notamment, comme illustré à la figure 14, configurer l'applicateur de telle sorte que la partie sécable 22 puisse être entièrement séparée du tube 21 après avoir exercé manuellement un mouvement de cassure en tenant le tube 21 d'une main et la partie sécable 22 entre deux doigts de l'autre main.

L'applicateur peut encore être configuré de manière à ce que la partie sécable 22 reste solidaire du tube 21 après l'utilisation, comme illustré sur la figure 15, par un pont de matière 29.

Lorsque l'extrémité sécable peut se détacher entièrement du tube, cela peut faciliter par exemple l'utilisation de l'applicateur à la manière d'une pipette, l'utilisateur pouvant obturer l'extrémité supérieure avec son doigt pour distribuer le produit, par exemple goutte-à-goutte, dans l'élément d'application.

Le tube de l'applicateur peut comporter un ou plusieurs produits de test.

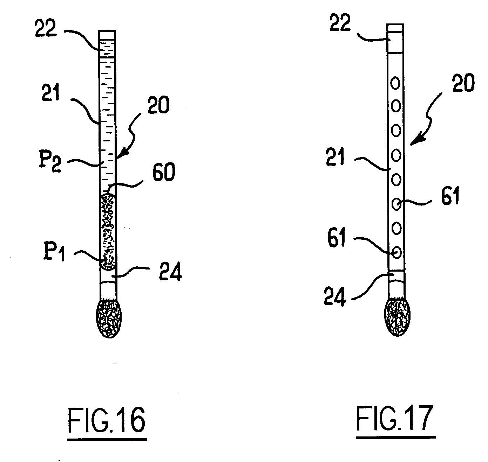

On a représenté à la figure 16 un tube comportant deux produits de test liquides $P_1$ et $P_2$ se présentant sous forme de deux phases occupant chacune une portion de la longueur du tube.

Le deux produits $P_1$ et $P_2$ sont au contact l'un de l'autre par une interface 60.

L'un des produits peut aussi se présenter sous la forme d'au moins un globule à l'intérieur de l'autre phase, par exemple sous forme de plusieurs globules 61 comme illustré à la figure 17. Cela peut permettre par exemple de réaliser un dosage en se servant du tube comme d'une pipette ou d'améliorer l'esthétique de l'applicateur.

Plusieurs liquides différents peuvent aussi être dispersés sous la forme de plusieurs globules dans une même phase. Les différents globules peuvent ainsi correspondre à des produits de test contenant des actifs différemment dosés et/ou de natures différentes.

Le produit $P_2$, lorsque contenu dans le tube, peut encore être solide, étant constitué par exemple par une poudre soluble dans le produit $P_1$, les produits $P_1$ et $P_2$ étant séparés par un bouchon avant l'utilisation.

Le volume de produit $P_2$ peut être suffisamment faible pour que le produit $P_2$ puisse se dissoudre facilement lors de l'utilisation.

Dans les exemples décrits précédemment, le tube comporte un canal intérieur unique mais on ne sort pas du cadre de la présente invention lorsque le tube comporte des canaux multiples.

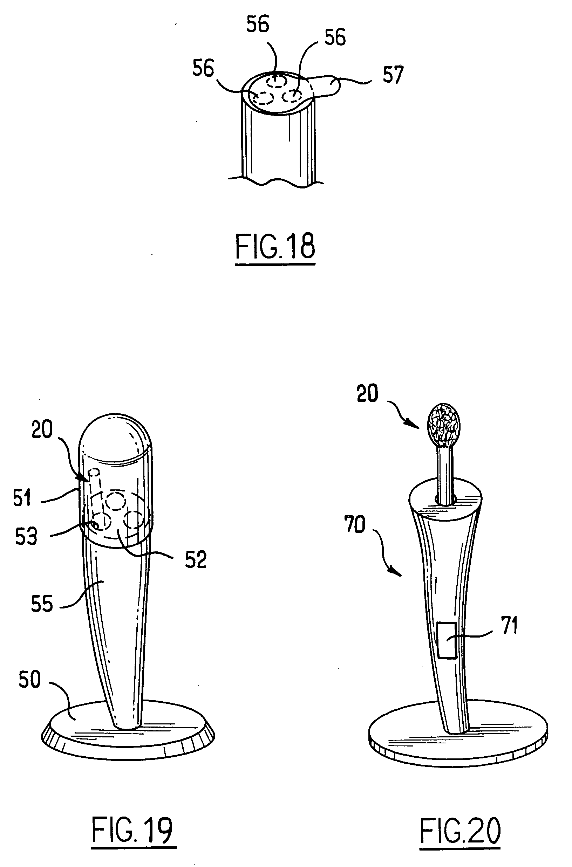

On a représenté à la figure 18 l'extrémité supérieure d'un tube comportant par exemple trois canaux intérieurs 56 contenant par exemple chacun un liquide et un bouchon associé, le tube pouvant être obturé à cette extrémité avant l'utilisation par un opercule amovible 57, lequel est par exemple collé ou thermosoudé sur le tube.

On a représenté à la figure 19 un récipient pouvant recevoir un applicateur avant ou après l'utilisation.

Un tel récipient peut par exemple comporter un socle 50 supportant un corps 55 dont l'extrémité supérieure est configurée pour permettre la fixation d'un capuchon de fermeture 51 permettant de fermer de manière sensiblement étanche le récipient, un élément de support 52 étant disposé à l'intérieur du corps 55 et comportant au moins un orifice 53 permettant d'y engager l'applicateur 20.

Ainsi, l'utilisateur peut, après une utilisation, s'il le souhaite, disposer l'applicateur à l'intérieur du récipient afin de le réutiliser plus tard. La présence du capuchon 51 permet d'éviter que l'élément d'application ne sèche, par exemple.

On peut encore utiliser, en association avec au moins un applicateur, un support 70 tel que représenté à la figure 20, permettant de maintenir l'applicateur avec l'élément d'application apparent, ce support pouvant par exemple comporter des moyens 71 permettant de rompre l'extrémité sécable alors que l'applicateur est en place dans le support 70. Ces moyens 71 comportent par exemple une fenêtre permettant d'accéder à l'extrémité sécable ou un élément mobile par rapport au support et dont l'actionnement provoque une poussée latérale sur l'extrémité sécable. Le tube peut encore être dépourvu d'extrémité sécable mais simplement comporter une extrémité fermée et le support 70 être équipé par exemple d'une lame ou d'une pointe permettant de couper ou de percer le tube pour permettre à l'air de pénétrer à l'intérieur et au liquide et au bouchon de s'évacuer lorsque l'applicateur est utilisé.

Bien entendu, l'invention n'est pas limitée aux exemples de réalisation qui viennent d'être décrits.

L'extrémité du tube 21 opposée à l'extrémité par laquelle le produit de test sort peut par exemple être fermée autrement qu'avec une partie sécable manuellement, au moyen d'un bouchon ou d'un piston, par exemple de l'une des manières représentées sur les figures 3 à 8 du brevet US 3 958 571, dont le contenu est incorporé ici par référence.

Le tube 21 peut être réalisé avec une partie renflée, permettant par exemple d'exercer une pression sur le produit pour lui faire quitter le tube.

Par « tube », il faut comprendre tout corps de préférence généralement allongé, de section constante ou non, présentant au moins un canal intérieur susceptible de contenir un liquide, un tel tube pouvant présenter un axe longitudinal rectiligne ou non. L'invention n'est pas limitée à un tube de section extérieure circulaire, ni à un tube réalisé conformément à l'enseignement du brevet US 5 702 035.

On peut tester la neurosensibilité cutanée d'un individu, comme décrit précédemment, ou bien encore tester tout type de sensibilité biologique, chimique, thermique ou mécanique de la peau, d'une muqueuse ou des phanères.

Dans toute la description, y compris les revendications, l'expression « comportant un » doit être comprise comme étant synonyme de « comportant au moins un », sauf si le contraire est spécifié.

**Revendications**

1. Kit (10) d'évaluation ou de diagnostic, comportant une pluralité d'applicateurs (20) contenant des produits de test de natures différentes et/ou comportant au moins un composé à des concentrations différentes, chaque applicateur (20) comportant :

    - un tube (21),
    - un bouchon d'un liquide ou d'une poudre (24) à l'intérieur du tube (21), et
    - au moins un produit de test contenu dans un espace intérieur du tube (21) délimité d'un premier côté par le bouchon de liquide ou de poudre (24), ce bouchon étant agencé pour s'évacuer avec le produit lorsque ce dernier quitte l'espace intérieur du tube lors de l'utilisation.

2. Kit selon la revendication 1, **caractérisé par le fait qu'**il comporte une pluralité d'applicateurs (20) contenant des produits de natures différentes.

3. Kit selon la revendication 1, **caractérisé par le fait qu'**il comporte une pluralité d'applicateurs (20) contenant des produits ($P_1$ ; $P_2$ ; $P_3$ ; $P_4$) comportant au moins un composé à des concentrations ($C_1$, $C_2$, $C_3$, $C_4$) différentes.

4. Kit selon la revendication 3, **caractérisé par le fait qu'**il comporte au moins deux produits comportant au moins un composé avec des concentrations variant d'un facteur d'au moins deux d'un applicateur (20) à un autre.

5. Kit selon l'une des revendications 3 et 4, **caractérisé par le fait qu'**au moins un produit contient un agent de stimulation du système nerveux périphérique.

6. Kit selon la revendication 5, **caractérisé par le fait que** l'agent de stimulation du système nerveux périphérique est choisi dans le groupe constitué par : les capsaicinoids naturels ou synthétiques, de préférence la capsaïcine, l'homocapsaïcine, l'homodihydrocapsaïcine, la nordihydrocapsaïcine, la dihydrocapsaïcine ; l'acide lactique, l'acide glycolique, l'éthanol à une concentration supérieure à 50%, l'huile de moutarde.

7. Kit selon l'une des revendications 5 et 6, **caractérisé par le fait que** la concentration en agent de stimulation du système nerveux périphérique est comprise entre $10^{-6}$ et $10^{-2}$ % en poids.

8. Kit selon la revendication 2, **caractérisé par le fait que** les produits de test contiennent des allergènes de natures différentes.

9. Kit selon la revendication précédente, **caractérisé par le fait que** les allergènes sont choisis dans le groupe constitué par : les allergènes issus des acariens, des poils et squames d'animaux, des moisissures, des venins d'hyménoptères, des aliments, des métaux, notamment du nickel, du caoutchouc, de tout composé pouvant être rencontré dans des produits destinés à être appliqués sur le corps ou les cheveux.

10. Kit selon l'une quelconque des revendications précédentes, **caractérisé par le fait qu'**il comporte un boîtier (11) comportant des compartiments (12 ; 17) dans lesquels sont logés les applicateurs (20).

11. Kit selon la revendication précédente, **caractérisé par le fait que** le boîtier (11) comporte au moins un compartiment (12) configuré pour recevoir un unique applicateur (20).

12. Kit selon la revendication 10, **caractérisé par le fait que** le boîtier comporte au moins un compartiment (17) configuré pour recevoir une pluralité d'applicateurs (20).

13. Kit selon l'une quelconque des revendications 1 à 9, **caractérisé par le fait qu'**il comporte au moins un sachet (31 ; 33)) de conditionnement d'au moins un applicateur (20).

14. Kit selon la revendication précédente, **caractérisé par le fait qu'**il comporte un chapelet (30) de sachets (31) contenant chacun au moins un applicateur (20).

**15.** Kit selon l'une quelconque des revendications précédentes, **caractérisé par le fait que** chaque applicateur (20) comporte au moins une indication (25) correspondant à la nature du produit de test contenu à l'intérieur du tube (21) et/ou à la concentration du composé contenu dans le produit de test.

**16.** Kit selon la revendication précédente, **caractérisé par le fait que** l'indication (25) comporte au moins un signe alphanumérique ou une couleur.

**17.** Kit selon l'une quelconque des revendications précédentes, **caractérisé par le fait que** le volume de produit de test contenu dans le tube (21) est compris entre 0,01 ml et 5 ml, de préférence 0,05 ml et 1 ml.

**18.** Kit selon la revendication 1, **caractérisé par le fait que** l'espace intérieur est délimité d'un second côté, opposé au premier, par une partie (22) sécable, amovible, perforable ou déformable.

**19.** Kit selon la revendication précédente, **caractérisé par le fait que** l'applicateur (20) comporte un élément de maintien de la partie sécable (22) sur l'applicateur après son sectionnement.

**20.** Kit selon l'une quelconque des revendications précédentes, **caractérisé par le fait que** le tube (21) est muni à une extrémité d'un élément d'application (23), cet élément d'application étant séparé du produit de test avant l'utilisation par le bouchon de liquide ou de poudre (24).

**21.** Kit selon la revendication 20, **caractérisé par le fait que** l'élément d'application est choisi dans le groupe constitué par : un embout en coton (23), un embout en mousse (35), une pointe feutre (37), un embout floqué (36), un embout en céramique ou en un matériau fritté.

**22.** Kit selon l'une quelconque des revendications 1 à 20, **caractérisé par le fait que** le tube (21) est dépourvu d'élément d'application.

**23.** Kit selon la revendication 22, **caractérisé par le fait que** le tube comporte une extrémité (28) configurée pour permettre d'effectuer une scarification de la peau.

**24.** Kit selon l'une quelconque des revendications précédentes, **caractérisé par le fait que** le bouchon comporte un liquide et **par le fait que** ce dernier est choisi dans le groupe constitué par : les huiles minérales, les produits fluorés, les silicones.

**25.** Kit selon l'une quelconque des revendications 1 à 23, **caractérisé par le fait que** le bouchon (24) comporte une poudre, et **par le fait que** cette dernière est choisie dans le groupe constitué par : les poudres de microsphères de copolymères, de Nylon®, des cires, des silices, et des silicones.

**26.** Procédé d'évaluation du niveau de neurosensibilité cutanée d'un individu, comportant les étapes suivantes :

1) appliquer sur une zone cutanée de l'individu un premier produit contenant un véhicule physiologiquement acceptable et un agent de stimulation du système nerveux périphérique, le produit étant appliqué en utilisant un applicateur (20) comportant un tube (21), un bouchon d'un liquide ou d'une poudre (24) à l'intérieur du tube, le produit étant contenu dans un espace intérieur du tube délimité d'un premier côté par le bouchon de liquide ou de poudre (24), ce bouchon étant agencé pour s'évacuer avec le produit lorsque ce dernier quitte l'espace intérieur du tube lors de l'utilisation,
2) recueillir une information représentative de la détection éventuelle par l'individu d'une sensation dysesthésique,
3) en l'absence de sensation détectée par l'individu, recommencer les étapes 1) et 2) avec un produit contenant une concentration plus élevée du même agent jusqu'à ce que l'individu détecte une sensation dysesthésique ou jusqu'à ce qu'un produit ayant une valeur maximale de concentration ($C_4$) dudit agent soit appliqué,
4) déduire, de la dernière concentration appliquée, une information quant à la neurosensibilité cutanée de l'individu.

**27.** Procédé d'évaluation d'un produit de traitement, comportant les étapes suivantes :

- réaliser les étapes du procédé selon la revendication 26,
- appliquer le produit de traitement,
- après un nombre d'applications donné et/ou qu'une durée déterminée se soit écoulée, réaliser à nouveau les étapes du procédé selon la revendication 26,
- évaluer l'efficacité du traitement suivi.

FIG.1

FIG.2

FIG.3

FIG.4

FIG.6

FIG.5

FIG.7

FIG.8

FIG.9

FIG.10

FIG.11

FIG.12

FIG.13

FIG.14

FIG.15

FIG.16

FIG.17

FIG.18

FIG.19

FIG.20

| | RAPPORT PARTIEL DE RECHERCHE EUROPEENNE | Numéro de la demande |
|---|---|---|
| Office européen des brevets | qui selon la règle 45 de la Convention sur le brevet européen est consideré, aux fins de la procédure ultérieure, comme le rapport de la recherche européenne | EP 03 29 2399 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 553 534 A (TSAO CHIEN HUA) 4 août 1993 (1993-08-04) * colonne 5, ligne 39 - colonne 6, ligne 58; figures 13,14 * --- | 1-25 | A61M35/00 |
| Y | DE 29 20 213 A (ZDARSKY EDUARD) 20 novembre 1980 (1980-11-20) * abrégé; figures * --- | 1-25 | |
| Y | US 5 490 736 A (SMEDLEY WILLIAM H ET AL) 13 février 1996 (1996-02-13) * le document en entier * --- | 1-25 | |
| X | WO 98 34581 A (CAO JIANHUA) 13 août 1998 (1998-08-13) * abrégé; figures * --- | 1 | |
| Y | US 5 143 210 A (WARWICK S JOHN ET AL) 1 septembre 1992 (1992-09-01) * le document en entier * --- | 1-25 | DOMAINES TECHNIQUES RECHERCHES (Int.Cl.7) |
| Y | FR 607 405 A (WITTE OTTO) 2 juillet 1926 (1926-07-02) * le document en entier * --- -/-- | 1-25 | A61M B65D |

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

26,27

Raison pour la limitation de la recherche:

Article 52 (4) CBE - Méthode de diagnostic appliquée au corps humain ou animal

| Lieu de la recherche | Date d'achèvement de la recherche | Examinateur |
|---|---|---|
| MUNICH | 19 novembre 2003 | Ehrsam, F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
-----------------
& : membre de la même famille, document correspondant

EPO FORM 1503 03.82 (P04C08)

Office européen
des brevets

**RAPPORT PARTIEL
DE RECHERCHE EUROPEENNE**

Numero de la demande

EP 03 29 2399

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| A | WO 89 10156 A (NUGENT WILLIAM MICHAEL ;WOLFSON HARRIS PHILLIP (US)) 2 novembre 1989 (1989-11-02) * figure 7 *<br>--- | 1 | |
| Y | US 5 152 742 A (SIMPSON IAN) 6 octobre 1992 (1992-10-06) * figures 2,3 *<br>--- | 1 | |
| Y | FR 1 408 584 A (SCHNEIDER HELMUT) 13 août 1965 (1965-08-13) * figure 2 *<br>--- | 1 | |
| Y | US 6 343 717 B1 (ZHANG JACK YONGFENG ET AL) 5 février 2002 (2002-02-05) * colonne 5, ligne 44 - ligne 59; figures *<br>--- | 1-25 | DOMAINES TECHNIQUES RECHERCHES (Int.Cl.7) |
| A | US 1 607 768 A (HANS MEUSEL) 23 novembre 1926 (1926-11-23) * figures *<br>--- | 1 | |
| A | US 5 702 035 A (TSAO CHIEN-HUA) 30 décembre 1997 (1997-12-30) * le document en entier *<br>--- | 1-15 | |
| A,D | US 3 958 571 A (BENNINGTON WILLIAM E) 25 mai 1976 (1976-05-25) * le document en entier *<br>----- | | |

EPO FORM 1503 03.82 (P04C11)

## ANNEXE AU RAPPORT DE RECHERCHE EUROPEENNE
## RELATIF A LA DEMANDE DE BREVET EUROPEEN NO.

EP 03 29 2399

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-11-2003

| Document brevet cité au rapport de recherche | | Date de publication | Membre(s) de la famille de brevet(s) | | Date de publication |
|---|---|---|---|---|---|
| EP 0553534 | A | 04-08-1993 | AU | 2095292 A | 29-07-1993 |
| | | | CA | 2075758 A1 | 29-07-1993 |
| | | | CN | 2157140 U | 23-02-1994 |
| | | | EP | 0553534 A1 | 04-08-1993 |
| | | | ZA | 9205972 A | 28-04-1993 |
| DE 2920213 | A | 20-11-1980 | DE | 2920213 A1 | 20-11-1980 |
| US 5490736 | A | 13-02-1996 | AUCUN | | |
| WO 9834581 | A | 13-08-1998 | WO | 9834581 A1 | 13-08-1998 |
| | | | AU | 1588697 A | 26-08-1998 |
| US 5143210 | A | 01-09-1992 | AUCUN | | |
| FR 607405 | A | 02-07-1926 | AUCUN | | |
| WO 8910156 | A | 02-11-1989 | AU | 3553589 A | 24-11-1989 |
| | | | WO | 8910156 A1 | 02-11-1989 |
| US 5152742 | A | 06-10-1992 | AUCUN | | |
| FR 1408584 | A | 13-08-1965 | AUCUN | | |
| US 6343717 | B1 | 05-02-2002 | AU | 1329102 A | 03-06-2002 |
| | | | BR | 0108028 A | 05-11-2002 |
| | | | CA | 2398919 A1 | 30-05-2002 |
| | | | CN | 1395535 T | 05-02-2003 |
| | | | EP | 1252075 A1 | 30-10-2002 |
| | | | WO | 0242175 A1 | 30-05-2002 |
| US 1607768 | A | 23-11-1926 | AUCUN | | |
| US 5702035 | A | 30-12-1997 | AUCUN | | |
| US 3958571 | A | 25-05-1976 | AUCUN | | |

EPO FORM P0460

Pour tout renseignement concernant cette annexe : voir Journal Officiel de l'Office européen des brevets, No.12/82